# EUROPEAN PATENT SPECIFICATION

(11) **EP 3 527 296 B1**
(45) Date of publication and mention of the grant of the patent: **19.07.2023**
(21) Application number: 17876787.7
(22) Date of filing: 28.09.2017
(51) Int. Cl.: B09B 3/00, B09B 5/00, B29B 17/02, C02F 1/28, C02F 3/00, C12P 7/10, C12P 7/56, C12P 19/02, H01M 8/06, H01M 8/16, B29K 1/00, B29L 31/48

(54) **METHOD FOR RECYCLING USED ABSORBENT ARTICLES**
RECYCLING-VERFAHREN FÜR GEBRAUCHTE SAUGFÄHIGE ARTIKEL
PROCÉDÉ DE RECYCLAGE D'ARTICLES ABSORBANTS USAGÉS

(30) Priority: 02.12.2016 JP 2016235415
(43) Date of publication of application: 21.08.2019
(73) Proprietor: Unicharm Corporation, Shikokuchuo-shi, Ehime 799-0111 (JP)
(72) Inventor: KONISHI, Takayoshi, Kanonji-shi Kagawa 769-1602 (JP); YAMAKI, Koichi, Kanonji-shi Kagawa 769-1602 (JP); HIRAOKA, Toshio, Kanonji-shi Kagawa 769-1602 (JP); KAMEDA, Noritomo, Kanonji-shi Kagawa 769-1602 (JP)
(74) Representative: Dolleymores
(86) International application number: PCT/JP2017/035333
(87) International publication number: WO 2018/100865

(56) References cited:
- WO-A1-2016/103985
- WO-A1-2016/103985
- JP-A- 2013 202 021
- JP-A- 2013 202 021
- JP-A- 2015 182 246
- JP-A- 2016 123 973

## Description

### FIELD

The present invention relates to a recycling method for used absorbent articles. More specifically, it relates to a recycling method for absorbent articles such as used paper diapers that contain human waste.

### BACKGROUND

Attempts have been made in the past to recycle used paper diapers. Japanese Unexamined Patent Publication No. 2003-200147 (PTL 1), for example, discloses a method for recovering hygienic materials from fouled sanitary products. In this method, the fouling-adhered sanitary products are cut into strips, the water absorbent paper cut into strips and the fouling-adhered plastic materials are separated from the water-absorbing materials, the separated water absorbent paper and fouling-adhered plastic materials are pulverized and the pulverized product is sorted in an air stream, thereby separating the water-absorbing materials comprising the combination of water absorbent paper and pulverized fouling-adhered plastic materials, into the water absorbent paper and the fouling-adhered plastic materials, and recovering the plastic material-sorted product obtained by separation of the water-absorbing materials, while the water-absorbing materials that have been combined with the separated plastic materials are mixed with the non-wetted sorted product from the water-absorbing materials that has been separated by sorting of the water-absorbing materials separated from the sanitary product, in an air stream, and the water-absorbing material mixture is sorted in the air stream to recover the non-wetted cotton pulp-rich sorted product in the water-absorbing material mixture.

Japanese Unexamined Patent Publication No. 2003-225645 (PTL 2) discloses a method of separating and recovering pulp components and water-absorbent polymers from used absorbent articles, at high yield and high purity to allow their reuse. In this method, a transition metal salt alone or a mixture of a transition metal salt with an alkali metal salt or an alkaline earth metal salt is added to a gelatinous mixture of a pulp component and water-absorbent polymer present in a used absorbent article, to dehydrate the moisture in the water-absorbent polymer and cause shrinkage and solidification of the water-absorbent polymer, while also coloring the water-absorbent polymer with the transition metal salt, after which the pulp component and water-absorbent polymer are separated and recovered.

### [CITATION LIST]

### [PATENT LITERATURE]

[PTL 1] Japanese Unexamined Patent Publication No. 2003-200147
[PTL 2] Japanese Unexamined Patent Publication No. 2003-225645
WO 2016/103985 A1 and JP 2016 123973 A relate to a method for recycling used absorbent articles using an ozone treatment step. At least one type of material constituting the absorbent articles is collected and recycled.

### SUMMARY

### [TECHNICAL PROBLEM]

The conventional recycling methods represented by PTL 1 and PTL 2 are merely material recycling, with focus on allowing recovery of high value-added materials, whereas the processing rate has been less than satisfactory.

It is an object of the present invention to provide a total recycling system for used paper diapers and the like with a high processing rate, based on not only recycling of materials but also recovery of portions thereof as energy.

### [SOLUTION TO PROBLEM]

As a result of much ardent research on recycling of used paper diapers and other materials containing human waste, the present inventors have completed this invention upon finding that by combining energy recovery with material recycling during recycling of human waste in used paper diapers and the like, it is possible to achieve a processing rate of 90 mass% or greater.

Specifically, the invention relates to a method of recycling used absorbent articles containing human waste, the absorbent article comprising constituent materials including pulp fibers and superabsorbent polymer, the method including:
a first step of separating the pulp fiber from the used absorbent articles containing human waste, to obtain regenerated pulp fiber, the first step generating waste water containing organic material,
a second step of conversion to solid fuel from the constituent materials of the absorbent articles remaining after separation of the pulp fiber, to obtain solid fuel, and
a third step of supplying the waste water containing organic material generated from the first step to a microbial fuel cell to obtain electric power while purifying the waste water,
wherein the processing rate for the used absorbent articles containing human waste is 90 mass% or greater, the processing rate being ((loaded amount of the used absorbent article containing human waste) - (final amount of solid waste treated)) / (loaded amount of the used absorbent article containing human waste) × 100, and wherein the method further includes a step of recovering the superabsorbent polymer.

The invention encompasses the following aspects.
[1] A method of recycling a used absorbent article containing human waste, the absorbent article comprising constituent materials including pulp fibers and superabsorbent polymer, the method including:
   a first step of separating the pulp fiber from the used absorbent article containing human waste, to obtain regenerated pulp fiber, the first step generating waste water containing organic material,
   a second step of conversion to solid fuel from the constituent materials of the absorbent articles remaining after separation of the pulp fiber, to obtain solid fuel, and
   a third step of supplying the waste water containing organic material generated from the first step to a microbial fuel cell to obtain electric power while purifying the waste water,
   wherein a processing rate for the used absorbent article containing human waste is 90 mass% or greater, the processing rate being ((loaded amount of the used absorbent article containing human waste) - (final amount of solid waste treated)) / (loaded amount of the used absorbent article containing human waste) × 100, and wherein the method further includes a step of recovering the superabsorbent polymer.
[2] The method according to [1], further including a fourth step of recovering a nutrient salt derived from the human waste.
[3] The method according to [1] or [2], further including a fifth step of supplying waste water containing pulp fiber that could not be separated out after separation of the pulp fiber in the first step, to a saccharification tank to obtain glucose.
[4] The method according to [3], further including a sixth step of fermenting the glucose obtained in the fifth step to obtain ethanol or lactic acid.
[5] The method according to any one of [1] to [4], further including, before the first step, a pretreatment washing step in which the used absorbent articles are washed.
[6] The method according to [5], wherein water discharged from the microbial fuel cell is returned to the pretreatment washing step.
[7] The method according to any one of [1] to [6], wherein the electric power obtained by the third step is supplied to at least one step from among the first step, second step, fourth step, fifth step, sixth step, step of recovering superabsorbent polymer and pretreatment washing step.
[8] The method according to any one of [1] to [7], wherein the solid fuel obtained by the second step is used as fuel for operation of at least one step from among the first step, third step, fourth step, fifth step, sixth step, step of recovering superabsorbent polymer and pretreatment washing step.
[9] The method according to any one of [1] to [8], wherein an amount of waste material generated by the method is less than 10 mass% of an amount of used absorbent articles that have been treated.
[10] The method according to any one of [1] to [9], wherein a throughput ratio in the first step, second step and third step is changed according to a demand for a purpose of reuse.

### [ADVANTAGEOUS EFFECTS OF INVENTION]

According to the invention it is possible to recycle used absorbent articles containing human waste at a high processing rate.

### BRIEF DESCRIPTION OF DRAWINGS

FIG. 1 is a flow chart for an embodiment of the method of the invention.
FIG. 2 is a flow chart for another embodiment of the method of the invention.
FIG. 3 is a flow chart for yet another embodiment of the method of the invention.
FIG. 4 shows examples of applied uses of recycled materials.
FIG. 5 shows an example of the construction of a microbial fuel cell.

### DESCRIPTION OF EMBODIMENTS

The present invention is a method of recycling used absorbent articles containing human waste.

The absorbent articles are not particularly restricted so long as they contain pulp fibers and superabsorbent polymer, and examples include paper diapers, urine-absorbing pads, bed sheets, sanitary napkins and pet sheets.

Absorbent articles are usually composed of constituent materials such as pulp fibers, superabsorbent polymer, nonwoven fabrics, thermoplastic resin films, rubber yarn and hot-melt adhesive.

There are no particular restrictions on the pulp fibers, and examples include fluffy pulp fibers and chemical pulp fibers.

A superabsorbent polymer, also known as SAP, has a three-dimensional network structure with an appropriately crosslinked water-soluble polymer, and it absorbs a few hundred to a few thousand times its weight in water while being essentially water-insoluble and preventing absorbed water from emerging even with some degree of pressure application, with examples thereof including starch-based, acrylic acid-based and amino acid-based particulate or fibrous polymers.

The method of the invention includes the following steps.
(1) A first step of separating pulp fiber from used absorbent articles containing human waste, to obtain regenerated pulp fiber.
(2) A second step of conversion to solid fuel from the constituent materials of the absorbent articles remaining after separation of the pulp fiber, to obtain solid fuel.
(3) A third step of supplying waste water containing organic material generated from the first step to a microbial fuel cell to obtain electric power while purifying the waste water.
(4) A step of recovering superabsorbent polymer.

The method of the invention may also include the following step.
(4) A fourth step of recovering nutrient salts derived from the human waste.
(5) A fifth step of supplying waste water containing pulp fiber that could not be separated out after separation of the pulp fiber in the first step, to a saccharification tank to obtain glucose.
(6) A sixth step of fermenting the glucose obtained in the fifth step to obtain ethanol or lactic acid.

The method of the invention may further include, before the first step, a pretreatment washing step in which the used absorbent articles are washed.

The first step is a step of separating pulp fiber from used absorbent articles containing human waste, to obtain regenerated pulp fiber. The first step may also be referred to hereunder as "pulp fiber regeneration step".

The method for the first step is not limited so long as the pulp fiber can be separated from the used absorbent articles containing human waste to obtain regenerated pulp fiber, and the following method may be mentioned as an example.

By agitating used absorbent articles containing human waste in an aqueous solution containing an inactivating agent that is able to inactivate superabsorbent polymers, the used absorbent articles are decomposed into constituent materials, the superabsorbent polymers are inactivated, and the human waste is dissolved or dispersed in the aqueous solution. After completing agitation, the solid-liquid mixture is passed through a sieve and separated into a first fraction containing nonwoven fabric, thermoplastic resin film and rubber yarn materials, and a second fraction containing aqueous solution, pulp fiber, inactivated superabsorbent polymer and human waste materials.

The used absorbent articles containing human waste may be subjected to pretreatment washing before decomposition into the constituent materials, removing out the human waste adhering to the outer surfaces of the used absorbent articles. By agitating the used absorbent articles from which the human waste adhering to the outer surfaces have been removed, in an aqueous solution containing an inactivating agent that is able to inactivate superabsorbent polymers, the used absorbent articles are decomposed into constituent materials, and the superabsorbent polymers are inactivated. After completing agitation, the solid-liquid mixture is passed through a sieve and separated into a first fraction containing nonwoven fabric, thermoplastic resin film and rubber yarn materials, and a second fraction containing aqueous solution, pulp fiber, inactivated superabsorbent polymer and urine components. When pretreatment washing has been carried out, the water used for the pretreatment washing is treated as waste water; however, most of the solid portion of the human waste adhering to the outer surfaces of the used absorbent articles is transferred to the waste water side, while most of the urine components absorbed in the absorbent body migrate into the second fraction.

The pulp fibers are then separated and recovered from the second fraction. There is no limitation to the method of separating and recovering the pulp fibers from the second fraction; however, examples include a method of utilizing the difference in specific gravity for sedimentation separation in water, a method of separation by passing through a sieve having a prescribed mesh opening, and a method of separation with a cyclone centrifuge. For example, suitable agitation of the second fraction causes pulp fiber to float in the aqueous solution, and skimming off the floating pulp fiber with a sieve or the like allows the pulp fiber to be separated and recovered. Low quality regenerated pulp fiber is obtained by the steps up to this stage.

The separated and recovered pulp fiber may then be further disinfected, washed, dewatered and dried to obtain high quality regenerated pulp fiber.

The inactivating agent that can inactivate superabsorbent polymers may include, but is not limited to, organic acids, calcium compounds (hydrated lime, calcined lime, calcium chloride), magnesium sulfate, magnesium chloride, aluminum sulfate and aluminum chloride, among which organic acids and calcium compounds are preferred.

An organic acid is unlikely to damage the pulp fibers, and therefore using an organic acid allows recovery of pulp fibers with low damage, i.e. pulp fibers with a high water absorption factor and high water retention factor. When an organic acid is used as the inactivating agent, the pH of the organic acid aqueous solution is limited to no higher than 2.5. In other words, the aqueous solution containing the inactivating agent that can inactivate the superabsorbent polymer is an organic acid aqueous solution having a pH of preferably no higher than 2.5.

Organic acids include citric acid, tartaric acid, glycolic acid, malic acid, succinic acid, acetic acid and ascorbic acid, with citric acid being particularly preferred. The chelating effect of citric acid traps metal ions and the like present in human waste, allowing their removal, and the washing effect of citric acid can potentially provide a high fouling component-removal effect.

The pH of the organic acid aqueous solution is no higher than 2.5, preferably 1.3 to 2.4 and more preferably 1.5 to 2.1. If the pH is too high, it may not be possible to sufficiently lower the water-absorbing capacity of the superabsorbent polymer. If the pH is too low, the risk of corrosion of the equipment will increase, lowering its service life, and large amounts of alkaline chemicals may be necessary for neutralizing treatment during waste water treatment.

The pH will vary depending on the water temperature; however, the pH for the purpose of the invention is the pH measured at an aqueous solution temperature of 20°C.

The organic acid concentration of the organic acid aqueous solution is not restricted so long as the pH of the organic acid aqueous solution is no higher than 2.5; however, when the organic acid is citric acid, the citric acid concentration is preferably 1 mass% or greater, more preferably 2.0 to 4.0 mass% and even more preferably 2.0 to 3.0 mass%. For sterilization, a concentration of 2 mass% or greater is preferred.

The method of disinfecting the separated and recovered pulp fibers is not restricted, and it may be carried out by immersion of the pulp fibers in an aqueous solution dissolving a disinfectant such as sodium hypochlorite or chlorine dioxide, or ozone water, electrolytic water (acidic electrolytic water) or the like. From the viewpoint of economy and general utility, a method using ozone water or a sodium hypochlorite aqueous solution is preferred.

The method of washing the pulp fibers is not limited, but in the case of a batch process, water is used at 500 to 5000 parts by mass to 100 parts by mass of pulp fibers (dry mass), and washing is carried out for 3 to 60 minutes.

The method of dewatering the pulp fibers is not limited and may be 1 to 10 minutes of dewatering using a dehydrator, for example.

The method for drying the pulp fiber is also not limited, and it may be carried out using a drier such as a hot air drier, for example.

The drying conditions are not particularly restricted so long as the pulp fiber is sufficiently dried, and an example is a drying temperature of 100 to 200°C and a drying time of 10 to 120 minutes.

The moisture content of the dried pulp fiber is preferably 5 to 13 mass%. If the moisture content is too low, the hydrogen bonds may become too strong resulting in excessive hardness, while conversely if the moisture content is too high, growth of mold and the like may occur.

The first step may be carried out by the following method.

The used absorbent articles containing human waste are subjected to pretreatment washing, and then open holes or cuts are formed in the front sheets or back sheets of the washed absorbent articles, the contents of the absorbent articles (pulp fibers and superabsorbent polymer) are discharged using a roll press, and the discharged water-containing pulp fibers and superabsorbent polymer are subjected to inactivation treatment to inactivate the superabsorbent polymer, after which the pulp fibers are separated.

The pretreatment washing is not restricted and may be immersion in water at 50°C or higher and below 100°C, and agitation for 1 minute or longer. The waste water containing the human waste generated by pretreatment washing is supplied to the third step.

The production volume of low quality regenerated pulp fiber, medium quality regenerated pulp fiber and high quality regenerated pulp fiber can be changed according to the amount of human waste in the used absorbent articles and the demand for regenerated pulp fibers. By changing their production volume, it is possible to minimize the amount of final waste material and increase the recycling efficiency.

Since pulp is a wood resource, this will reduce CO₂ emission. The immobilized CO₂ may be directly recovered and reused, thus contributing to CO₂ reduction.

The second step is a step of conversion to solid fuel from the constituent materials of the absorbent articles remaining after separation of the pulp fiber, to obtain solid fuel. This second step may also be referred to hereunder as "solid fuel conversion step".

The phrase "constituent materials of absorbent articles remaining after separation of pulp fiber" may also be referred to hereunder as "nonwoven fabric and film components". The nonwoven fabric and film components are, for example, the first fraction of the first step, or the residue remaining after using a roll press in the first step to discharge the contents of the absorbent articles. The nonwoven fabric and film components include nonwoven fabric, thermoplastic resin film and rubber yarn components, but may also include inactivated superabsorbent polymers.

Solid fuel conversion is also known as RPF (Refuse Paper & Plastic Fuel) conversion, and a common method may be employed.

The obtained solid fuel can be used as fuel for operation of other steps in the method of the invention.

The third step is a step of supplying waste water containing organic material generated from the first step to a microbial fuel cell to obtain electric power while purifying the waste water. This third step may also be referred to hereunder as "microbial fuel cell step".

A microbial fuel cell is a device that utilizes microbes to convert organic material fuel to electrical energy. In a microbial fuel cell, a negative electrode and positive electrode are immersed in a solution of organic material fuel and the electrons generated upon oxidative decomposition of the organic material at the negative electrode by microbes are collected, the electrons then migrating toward the positive electrode through an external circuit, and the electrons being consumed at the positive electrode by reduction reaction of an oxidizing agent. The electrons flow by the difference in oxidation-reduction potential of the chemical reaction at the negative electrode and the chemical reaction at the positive electrode, and energy corresponding to the product of the difference in potential of both electrodes and the current flowing through the external circuit is obtained in the external circuit.

In the third step, the waste water containing organic materials is loaded into the microbial fuel cell to lower the TOC concentration of the waste water while recovering electric power by electricity generation. In the microbial fuel cell, organic materials such as human waste and fine pulp in the waste water are oxidatively decomposed by microbes, reducing the TOC concentration of the waste water and generating electricity.

The microbes used in the microbial fuel cell are not particularly restricted so long as they can contribute to oxidative decomposition of the organic materials while generating electrical energy; however, hydrogen-producing microbes are used as a rule, among which obligatory anaerobes and facultative anaerobic bacteria are preferably used.

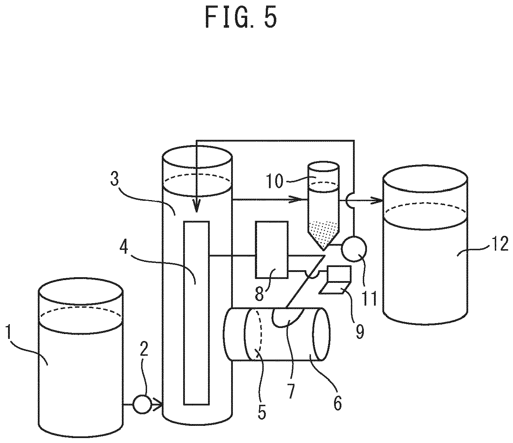

Fig. 5 shows an example of the configuration of a microbial fuel cell to be used for the invention. In the drawing, 1 is a waste water tank, 2 is a pump, 3 is a negative electrode reaction tank, 4 is a negative electrode, 5 is a proton-exchange membrane, 6 is a positive electrode tank, 7 is a positive electrode, 8 is a tester, 9 is a personal computer, 10 is a sludge settling tank, 11 is a pump and 12 is a purification tank.

The pH of the waste water from the microbial fuel cell is preferably lower than 8.0. If the pH of the waste water from the microbial fuel cell is too high, the generation efficiency of the microbial fuel cell will be reduced.

The waste water loaded into the microbial fuel cell preferably has a TOC concentration of no greater than 10,000 mg/L. The waste water from the microbial fuel cell preferably has a TOC concentration of no greater than 160 mg/L. Each local government has different ordinances; however, under the ordinances of Kagawa Prefecture, for example, waste water with a TOC concentration of up to 160 mg/L can be directly discharged into rivers. A TOC concentration of 30 mg/L or lower is more preferred for reuse as water for recycling treatment. If the TOC concentration falls below 30 mg/L, the amount of organic material that can be consumed by the microbes in the microbial fuel cell will be reduced, often lowering the generation efficiency.

If the TOC concentration is no greater than 30 mg/L in the waste water from the microbial fuel cell, then the waste water can be used in the pretreatment washing step in which the used absorbent article is washed, before the first step.

In the microbial fuel cell step, solid sludge is generated due to accumulation of non-decomposed organic material and dead microbes. In the method of the invention, the solid sludge is waste material that is to be disposed of without recycling. Specifically, in the method of the invention, the waste material generated from the used absorbent articles containing human waste essentially consists only of the solid sludge generated in the microbial fuel cell step.

When a calcium compound has been used for inactivation of the superabsorbent polymer, calcium ion will be present in the waste water supplied to the microbial fuel cell. The calcium ion can increase the generation efficiency of the microbial fuel cell.

Waste water is usually treated with microbes in a clarification tank; however, such waste water treatment uses electricity and incurs cost. The present invention is therefore preferred as this is recovered as energy. Because a large amount of sludge is generated in a clarification tank with aerobic treatment, costs are also incurred for sludge treatment and the recycle efficiency is poor.

Carrying out the third step allows recovery of electric power by generation of electricity simultaneously with water purification from recycled waste water containing human waste, that conventionally has not been recycled and has been a cause of increased load in purification treatment, and it is thus possible to realize a complete recycling system for used absorbent articles. If the organic materials in waste water (such as human waste, chemicals, decomposed SAP and fine pulp fiber) are given as feed in an environment that allows microbes to be efficiently active, then the activity of the microbes can be increased and electric power can be recovered from the proliferated microbes, so that residual organic materials which have been treated as recycling residue in the past can also be recycled as electric power. Conventional microbe treatment has mostly been aerobic treatment, requiring supply of air by aeration; however, since a microbial fuel cell is anaerobic treatment, there is no need for aeration and running energy cost is cheap.

By the method of the invention it is possible to achieve a processing rate of 90 mass% or greater for used absorbent articles containing human waste. The processing rate referred to here is: (loaded amount of human waste-containing disposable paper diapers - final amount of solid waste treated) ÷ loaded amount of human waste-containing disposable paper diapers × 100.

The fourth step is a step of recovering nutrient salts derived from the human waste. This fourth step may also be referred to hereunder as "nutrient salt recovering step".

A nutrient salt is a salt containing nitrogen, phosphorus or potassium that can be used as a fertilizer, and more specifically it may be an ammonium salt or phosphate salt.

Waste water from the first step or third step will sometimes contain nutrient salts from human waste. The nutrient salt-containing waste water is supplied to the fourth step.

The method for recovering nutrient salts is not limited, and examples include a method for recovering phosphorus-containing nutrient salts by crystallizing the phosphorus in nutrient salt-containing waste water as hydroxyapatite (hereunder referred to as "HAP method"), and a method for recovering phosphorus- and/or nitrogen-containing nutrient salts by crystallizing the phosphorus and/or nitrogen in waste water as magnesium ammonium phosphate (hereunder referred to as "MAP method").

The HAP method is a method utilizing the crystallization phenomenon of hydroxyapatite (Ca₁₀(OH)₂(PO₄)₆) produced by reaction between PO₄³⁻, Ca²⁺ and OH⁻ in solution. The formula for this reaction is as follows.

10Ca²⁺ + 20H⁻ + 6PO₄³⁻ -> Ca₁₀(OH)₂(PO₄)₆

In the HAP method, Ca²⁺ and OH⁻ are added to a phosphorus-containing aqueous solution, which is contacted with seed crystals in a supersaturated state (metastable zone), crystallizing the hydroxyapatite on the seed crystal surfaces and allowing the phosphorus in the solution to be recovered. The seed crystals used may be phosphorus ore, bone coal or calcium silicate hydrate.

In the HAP method, Ca²⁺ and OH⁻ are added to a phosphorus-containing aqueous solution; however, since Ca²⁺ will already be present in the waste water of the first step if a calcium compound has been used for inactivation of the superabsorbent polymer in the first step, it will not always be necessary to add fresh Ca²⁺.

In this method, it is necessary for the Ca²⁺ concentration to be 5 millimole/liter or greater and the pH to be 8 or higher, and more preferably the Ca²⁺ concentration is 10 millimole/liter or greater and the pH is 9 or higher.

The MAP method is a method utilizing the crystallization phenomenon of magnesium ammonium phosphate (MgNH₄PO₄·6H₂O) produced by reaction between PO₄³⁻ and NH₄⁺, Mg²⁺ in solution. The formula for this reaction is as follows.

Mg²⁺ + NH₄⁺ + PO₄³⁻ + 6H₂O -> MgNH₄PO₄·6H₂O

In this method, preferably the Mg²⁺ concentration is 30 to 60 millimole/liter, and preferably the pH is 6.8 to 7.7.

The fourth step is preferably carried out before the microbial fuel cell step (third step). Specifically, the nutrient salts are recovered from the waste water after separation of the pulp fiber, and then the waste water is loaded into the microbial fuel cell. Since waste water from which the pulp has been separated has the highest phosphorus concentration and recovery efficiency, and phosphorus entering the microbial fuel cell results in adhesion of phosphorus at the positive electrode and lower generation efficiency, the yield is increased if this is prevented by recovering the phosphorus beforehand.

The recovered nutrient salts recovered in the fourth step may be utilized as fertilizer.

When a fourth step is provided, incidentally, it is preferred to not add an oxidizing agent that disintegrates the superabsorbent polymer before the step of separating the nutrient salt-containing waste water from the constituent materials such as used diapers. In recycling of used diapers, an oxidizing agent such as sodium hypochlorite is sometimes added for the purpose of sterilization or bleaching; however, the effect of the oxidizing agent causes disintegration and micronization of the skeletal structure of the superabsorbent polymer, making it more difficult to remove, and micronized superabsorbent polymer contaminates the precipitate obtained by the HAP method or MAP method, causing the concentration of hydroxyapatite or magnesium ammonium phosphate in the precipitate to be lowered. Not adding an oxidizing agent before the step of separating the nutrient salt-containing waste water from the constituent materials such as used diapers, will increase the possibility of allowing the inactivated superabsorbent polymer to be removed in the step of separating the nutrient salt-containing waste water from the constituent materials such as used diapers.

The fifth step is a step of supplying waste water containing pulp fibers that could not be separated out after separation of the pulp fibers in the first step, to a saccharification tank to obtain glucose. This fifth step may also be referred to hereunder as "saccharification step".

When the fifth step is to be carried out, preferably a pretreatment washing step in which the used absorbent articles are washed, is provided before the first step so that human waste adhering to the outer surfaces of the used absorbent articles is removed in advance, to minimize any human waste components in the waste water supplied to the fifth step.

In the fifth step, the waste water containing non-separated fine pulp fibers is mixed with an enzyme in a saccharification tank, for saccharification of the pulp fibers by the enzyme to obtain a glucose-containing saccharified solution.

The enzyme used is one including at least a cellulase. The cellulase is not particularly restricted, but it is preferred to use highly hydrolytic cellulases produced by *Trichoderma, Aspergillus* or *Acremonium* microbes, for example. Of these, *Acremonium* cellulases are most particularly preferred for use since they have been found to exhibit very high hydrolytic ability even in the presence of lignin. Cellulases include endoglucanases which randomly cleave cellulose chains, exoglucanases that free cellobiose from the ends of cellulose chains (cellobiohydrolase) and β-glucosidases that degrade cellobiose to glucose, and cellulases with different degrading mechanisms may also be used in combination.

The amount of cellulase used for saccharification is not particularly restricted and may be set as appropriate according to the capacity of the saccharification tank, the amount of substance to be treated and the activity of the cellulase used. More specifically, the amount of cellulase used is preferably 0.1 to 30 wt% with respect to the amount of pulp fibers. If the amount of cellulase added with respect to the amount of pulp fibers is too small, a longer time will be required for enzymatic treatment, resulting in performance issues. If the amount is too large, the saccharification rate will not significantly improve in relation to the amount of increased enzyme usage, which is disadvantageous from a cost perspective.

The solid concentration in the mixture of the pulp fibers, inactivated superabsorbent polymer and water in the saccharification step is not restricted so long as the saccharification proceeds, but it is preferably 1 to 50 wt% and more preferably 5 to 20 wt%.

The pulp fiber content in the mixture of the pulp fibers, inactivated superabsorbent polymer and water in the saccharification step may be appropriately set depending on the activity of the cellulase used, and while it is not particularly restricted it is preferably 0.5 to 25 wt% and more preferably 2.5 to 10 wt%.

The temperature and pH during the saccharification step may be appropriately set depending on the type of cellulase used; however, it must be set within a range so that the cellulase adequately functions and is not inactivated. For example, the temperature is preferably set in a range of 10 to 70°C, with the pH preferably at 3.0 to 8.0. The treatment time is preferably about 5 to 72 hours.

The sixth step is a step of fermenting the glucose obtained in the fifth step to obtain ethanol or lactic acid. This sixth step may also be referred to hereunder as "fermentation step".

For production of ethanol, the glucose may be fermented using yeast. More specifically, yeast may be added to the glucose-containing saccharified solution for fermentation, or the glucose may be separated from the glucose-containing saccharified solution, and the separated glucose subsequently fermented by yeast. The yeast to be used for ethanol fermentation is not particularly restricted, and any natural yeast or gene recombinant yeast may be used. *Saccharomyces* yeast are a typical example. The amount of yeast used for ethanol fermentation is not particularly restricted, and it may be appropriately determined based on the capacity of the fermenter, the amount of sugar solution and the activity of the yeast used. The temperature and pH for the fermentation step are not particularly restricted and may be appropriately set depending on the amount of sugar supplied to the fermentation and the type of yeast.

Instead of yeast, lactic acid-producing bacteria or the like may be used to obtain a chemical product such as lactic acid.

Saccharification and fermentation may also be carried out simultaneously in a single step. That is, in the saccharification and fermentation step, cellulase and yeast may be added to pulp fiber-containing waste water to saccharify the pulp fiber by the cellulase and produce glucose, after which the glucose may be fermented by the yeast to produce ethanol. This method allows production of ethanol from pulp fiber-containing waste water in the same treatment tank. Ethanol production utilizing an enzyme saccharification method may be carried out, for example, by adding a cellulase solution, prepared by dissolving cellulase powder in a solvent such as a buffering solution, to pulp fiber-containing waste water for saccharification treatment, and then adding yeast for ethanol fermentation.

The step of recovering superabsorbent polymer may also be referred to hereunder as "SAP recovering step".

Since the solid-liquid mixture remaining after separating out the pulp fiber from the second fraction of the first step includes inactivated superabsorbent polymer, a sieve having a mesh opening that does not allow passage of the inactivated superabsorbent polymer while allowing passage of other components may be used to separate and recover the inactivated superabsorbent polymer from the solid-liquid mixture. The separated and recovered inactivated superabsorbent polymer is subjected to regenerating treatment to restore its moisture-absorbing power. The method of regenerating treatment is not restricted, but as an example it may be carried out by treating the inactivated superabsorbent polymer with an aqueous alkali metal salt solution.

Alkali metal salts to be used include water-soluble salts of lithium, sodium, potassium, rubidium and cesium. Preferred alkali metal salts include sodium chloride, potassium chloride, sodium nitrate, potassium nitrate, sodium sulfate and potassium sulfate, among which sodium chloride and potassium chloride are preferred.

The amount of alkali metal salt is preferably 20 millimole or greater, more preferably 30 to 150 millimole and even more preferably 40 to 120 millimole, per 1 g (dry mass) of superabsorbent polymer. If the amount of alkali metal salt is too small, restoration of the moisture-absorbing power of the superabsorbent polymer may be insufficient. If the amount of alkali metal salt is too large, excess alkali metal ion will remain in the treatment solution without being incorporated into the superabsorbent polymer, resulting in waste of the alkali metal salt and increased treatment cost.

The concentration of the alkali metal salt in the aqueous alkali metal salt solution is not particularly restricted so long as it is a concentration allowing the polyvalent metal ion to be dissociated from the superabsorbent polymer; however, it is preferably 20 millimole/liter or greater, more preferably 30 to 150 millimole/liter and even more preferably 40 to 120 millimole/liter. If the concentration is too low, restoration of the moisture-absorbing power of the superabsorbent polymer will be insufficient. If the concentration is too high, excess alkali metal ion will remain in the treatment solution without being incorporated into the superabsorbent polymer, resulting in waste of the alkali metal salt and increased treatment cost.

The treatment time with the aqueous alkali metal salt solution is not particularly restricted so long as it is a sufficient time to cause dissociation of polyvalent metal ions from the superabsorbent polymer; however, it is preferably 1 hour or greater, more preferably 2 to 10 hours and even more preferably 4 to 8 hours. If the treatment time is too short, restoration of the moisture-absorbing power of the superabsorbent polymer will be insufficient. If the treatment time exceeds a certain length the amount of moisture absorption of the superabsorbent polymer will no longer increase, so that any treatment time exceeding that length will be meaningless.

The temperature of the aqueous alkali metal salt solution is not particularly restricted so long as it is a temperature allowing dissociation of the polyvalent metal ions from the superabsorbent polymer; however, it will usually be a temperature higher than 0°C and lower than 100°C. Although room temperature is adequate, heating may be carried out to increase the reaction rate. In the case of heating, it is preferably from room temperature to 60°C, more preferably from room temperature to 40°C, and even more preferably from room temperature to 30°C.

When the SAP recovering step is to be carried out, preferably an oxidizing agent that disintegrates the superabsorbent polymer is not added before carrying out the SAP recovering step. During recycling of a used diaper, an oxidizing agent such as sodium hypochlorite is sometimes added for the purpose of sterilization or bleaching; however, the oxidizing agent causes disintegration and micronization of the skeletal structure of the superabsorbent polymer, so that it becomes difficult to recover the superabsorbent polymer.

The SAP recovering step is preferably carried out after separating the pulp in the first step, and before the nutrient salt recovering step (fourth step). Specifically, the high-quality aqueous polymer is separated and recovered from the treated water after separating the pulp, after which the nutrient salt is separated and recovered. The pulp fibers have the largest sizes among the solid components (fiber lengths of approximately 1.5 to 3 mm), the inactivated SAP being the next largest at 100 µm and the nutrient salts being approximately 10 µm, and therefore separation and recovery are accomplished with screens in order from the largest.

The method of the invention may further include, before the first step, a pretreatment washing step in which the used absorbent articles are washed. Washing can partially break up the diapers by physical damage during washing, thus separating out the pulp, while removing the adhering excreta by washing to ensure hygiene during recovery of the nonwoven fabric and film components. The waste water containing the human waste generated by the pretreatment washing step may be supplied to the third step.

The water discharged from the microbial fuel cell may be returned to the pretreatment washing step for use as washing water.

The electric power obtained by the third step may be supplied to at least one step from among the first step, second step, fourth step, fifth step, sixth step, SAP recovering step and pretreatment washing step.

The solid fuel obtained by the second step may be used as fuel for operation of at least one step from among the first step, third step, fourth step, fifth step, sixth step, SAP recovering step and pretreatment washing step.

The ratio of throughput in the first step, second step and third step may be changed according to the demand for the purpose of reuse. When the method of the invention includes at least the fourth to sixth steps, the throughput ratio for at least one from among the first step, second step, third step, SAP recovering step and fourth to sixth steps, is preferably changed depending on the demand for the purpose of reuse. For example, if the demand for ethanol or lactic acid is greater than for pulp fiber, then it is preferred to reduce the amount of pulp separated in the first step, and increase the amount of pulp treated in the fifth step.

The throughput ratio for each step may be changed to allow a step of obtaining high-purity, high-quality pulp. By controlling the volume of product according to the demand for use, operation can be conducted while maintaining a high processing rate even with varying demand.

By changing the throughput ratios for the first step, second step and third step in the method of the invention according to the amount of human waste in the used absorbent articles, the amount of waste material generated by the method can be reduced to less than 10 mass% of the amount of used absorbent articles that are treated. The amount of waste material is the amount of solid sludge generated from the microbial fuel cell. The solid sludge generated from the microbial fuel cell also has a low amount of organic material residue, making it unsuitable for reuse as solid fuel.

When the method of the invention includes at least one step from among the fourth to sixth steps, the throughput ratio for the first step, second step, third step, SAP recovering step and at least one of the aforementioned steps may be changed according to the amount of human waste in the used absorbent articles, to further reduce the amount of waste material.

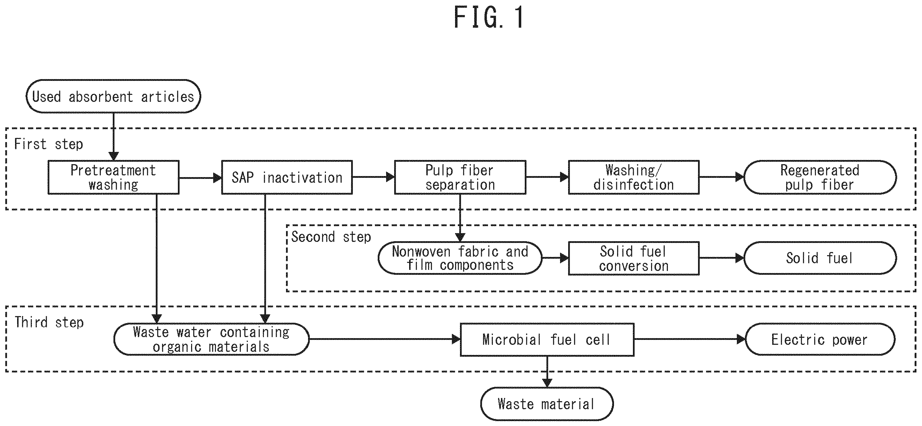

Fig. 1 shows a flow chart for an embodiment of the method of the invention.

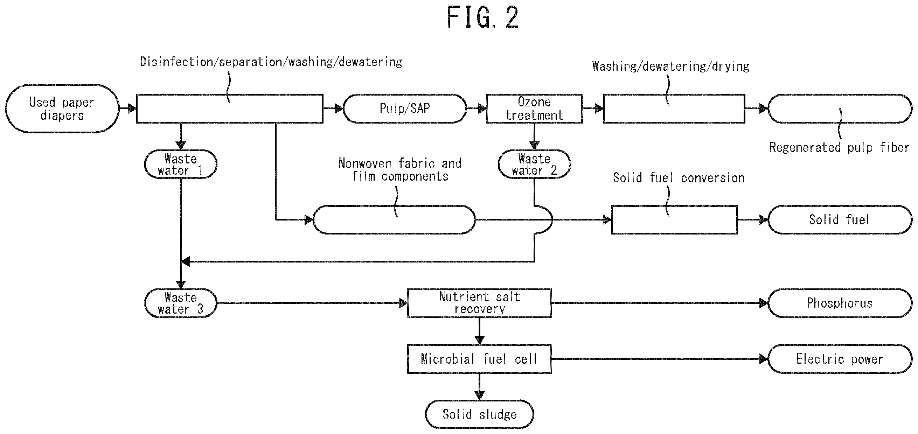

Fig. 2 shows a flow chart for another embodiment of the method of the invention.

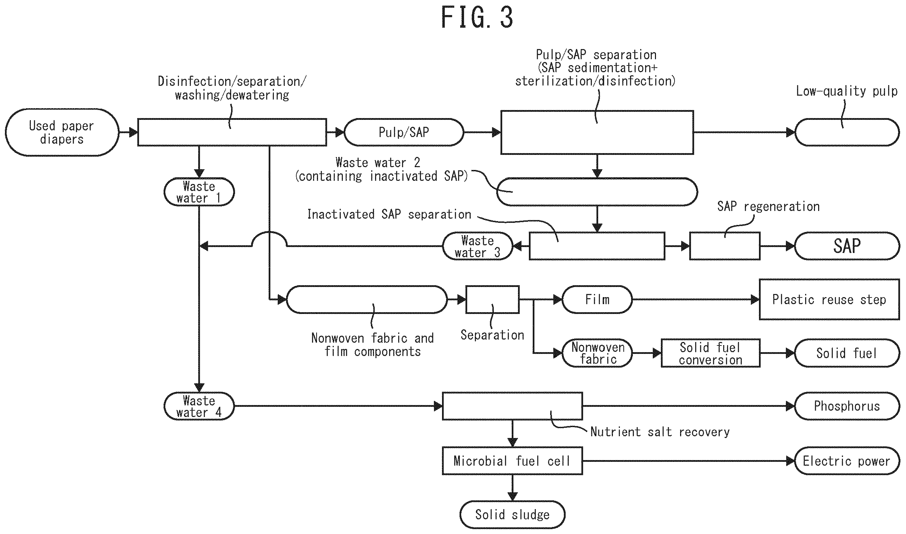

Fig. 3 shows a flow chart for yet another embodiment of the method of the invention.

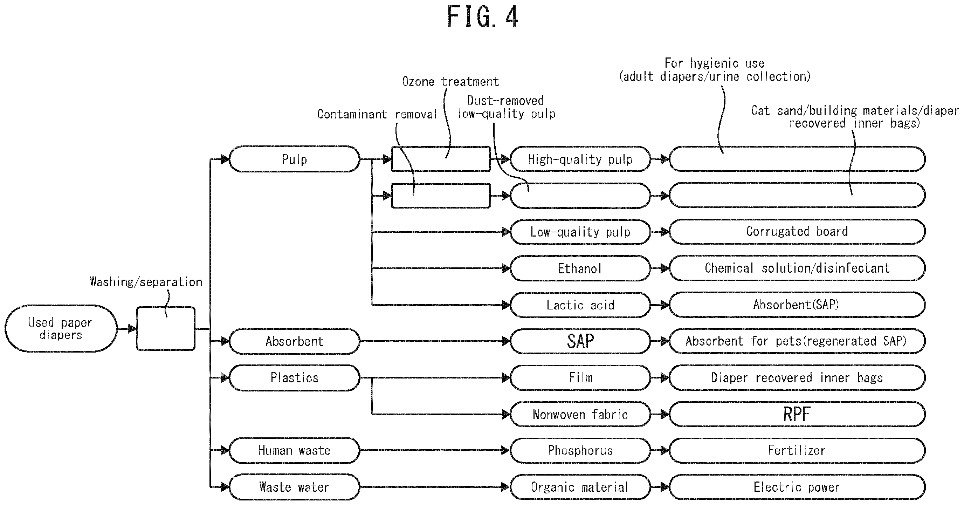

By using the method of the invention it is possible to produce recycled materials from used paper diapers in a purity and form suited for different applications, thereby permitting applications that are suited for different needs and conditions. Fig. 4 shows examples of applied uses of recycled materials.

### EXAMPLES

A 300 kg portion of human waste-containing disposable diapers was agitated in a treatment tank equipped with a stirrer, similar to a washing machine, to separate it into a fraction A including a small amount of pulp fiber, a small amount of superabsorbent polymer, human waste-derived residual solids, and plastics such as nonwoven fabric and film components, and a fraction "a" including pulp fiber, superabsorbent polymer and human waste components. The mass of fraction A was 129 kg. The amount of fraction "a" was 1.11 tons, which included (300 kg - 129 kg =) 171 kg of moisture present in the pulp fiber, superabsorbent polymer and human waste-containing disposable paper diapers, and organic materials from the human waste.

Solid-liquid separation of fraction "a" yielded regenerated pulp fiber. The mass of the recovered regenerated pulp fiber was 48 kg. Fraction A which included a small amount of pulp fiber, a small amount of superabsorbent polymer, human waste-derived residual solids and plastics such as nonwoven fabric and film components, was further agitated in a treatment tank equipped with a stirrer, similar to a washing machine, for further separation into the small amount of pulp fiber and the small amount of superabsorbent polymer, and the remainder was dried and compression molded to obtain solid fuel. The mass of the obtained solid fuel was 36 kg. Waste water "b" obtained by separating the small amount of pulp fiber and small amount of superabsorbent polymer had a mass of 30 kg.

Fraction "a" and waste water "b" were combined to obtain 1.14 tons of waste water "c". Waste water "c" included (171 kg + 30 kg =) 201 kg of moisture present in the human waste-containing disposable paper diapers, and organic materials from the human waste. The 1.14 tons of waste water "c" was introduced into a microbial fuel cell, and 95.7 kW of electricity was generated per day, resulting in production of 4 kg of solid sludge. In other words, the throughput for the microbial fuel cell was 201 kg - 4 kg = 197 kg.

Thus, the amount of the 300 kg of human waste-containing disposable paper diapers that could be treated was 48 kg + 36 kg + 197 kg = 281 kg, and the processing rate was 281 kg ÷ 300 kg × 100 ≈ 94 mass%.

### INDUSTRIAL APPLICABILITY

The method of the invention can be suitably used for efficient recycling of used absorbent articles containing human waste.

### REFERENCE SIGNS LIST

1 Waste water tank
2 Pump
3 Negative electrode reaction tank
4 Negative electrode
5 Proton-exchange membrane
6 Positive electrode tank
7 Positive electrode
8 Tester
9 Personal computer
10 Sludge settling tank
11 Pump
12 Purification tank

## Claims

1. A method of recycling a used absorbent article containing human waste, the absorbent article comprising constituent materials including pulp fibers and superabsorbent polymer, the method including:
a first step of separating the pulp fiber from the used absorbent article containing human waste, to obtain regenerated pulp fiber, the first step generating waste water containing organic material,
a second step of conversion to solid fuel from the constituent materials of the absorbent article remaining after separation of the pulp fiber, to obtain solid fuel, and
a third step of supplying the waste water containing organic material generated from the first step to a microbial fuel cell to obtain electric power while purifying the waste water,
wherein a processing rate for the used absorbent article containing human waste is 90 mass% or greater, the processing rate being ((loaded amount of the used absorbent article containing human waste) - (final amount of solid waste treated)) / (loaded amount of the used absorbent article containing human waste) × 100;
**characterized in that** the method further includes a step of recovering the superabsorbent polymer.

2. The method according to claim 1, further including a fourth step of recovering a nutrient salt derived from the human waste.

3. The method according to claim 1 or 2, further including a fifth step of supplying waste water containing pulp fiber that could not be separated out after separation of the pulp fiber in the first step, to a saccharification tank to obtain glucose.

4. The method according to claim 3, further including a sixth step of fermenting the glucose obtained in the fifth step to obtain ethanol or lactic acid.

5. The method according to any one of claims 1 to 4, further including, before the first step, a pretreatment washing step in which the used absorbent articles are washed.

6. The method according to claim 5, wherein water discharged from the microbial fuel cell is returned to the pretreatment washing step.

7. The method according to any one of claims 1 to 6, wherein the electric power obtained by the third step is supplied to at least one step from among the first step, the second step, the fourth step, the fifth step, the sixth step, the step of recovering superabsorbent polymer and the pretreatment washing step.

8. The method according to any one of claims 1 to 7, wherein the solid fuel obtained by the second step is used as fuel for operation of at least one step from among the first step, the third step, the fourth step, the fifth step, the sixth step, the step of recovering superabsorbent polymer and the pretreatment washing step.

9. The method according to any one of claims 1 to 8, wherein an amount of waste material generated by the method is less than 10 mass% of an amount of used absorbent articles that have been treated.

10. The method according to any one of claims 1 to 9, wherein a throughput ratio in the first step, the second step and the third step is changed according to a demand for a purpose of reuse.

## Patentansprüche

1. Verfahren für das Recycling eines benutzten absorbierenden Artikels, der menschliche Ausscheidungen enthält, wobei der absorbierende Artikel Materialbestandteile umfasst, die Zellstofffasern und superabsorbierendes Polymer einschließen, wobei das Verfahren Folgendes einschließt:
einen ersten Schritt der Abtrennung der Zellstofffaser aus dem benutzten absorbierenden Artikel, der menschliche Ausscheidungen enthält, um regenerierte Zellstofffaser zu erhalten, wobei der erste Schritt Abwasser erzeugt, das organisches Material enthält,
einen zweiten Schritt der Umwandlung zu Festbrennstoff aus den Materialbestandteilen des absorbierenden Artikels, die nach Abtrennung der Zellstofffaser verbleiben, um Festbrennstoff zu erhalten, und
einen dritten Schritt der Zuführung des organisches Material enthaltenden Abwassers, das in dem ersten Schritt erzeugt wurde, zu einer mikrobiellen Brennstoffzelle, um elektrischen Strom zu erhalten, während das Abwasser aufgereinigt wird,
wobei eine Verarbeitungsrate für den benutzten absorbierenden Artikel, der menschliche Ausscheidungen enthält, 90 Masse-% oder mehr beträgt, wobei die Verarbeitungsrate Folgendes ist: ((geladene Menge des benutzten absorbierenden Artikels, der menschliche Ausscheidungen enthält) - (Endmenge von behandeltem Feststoffabfall)) / (geladene Menge des benutzten absorbierenden Artikels, der menschliche Ausscheidungen enthält) × 100;
**dadurch gekennzeichnet, dass** das Verfahren weiter einen Schritt der Rückgewinnung des superabsorbierenden Polymers einschließt.

2. Verfahren nach Anspruch 1, das weiter einen vierten Schritt der Rückgewinnung eines Nährsalzes, das aus den menschlichen Ausscheidungen stammt, einschließt.

3. Verfahren nach Anspruch 1 oder 2, das weiter einen fünften Schritt der Zuführung von Abwasser, das Zellstofffaser enthält, die nach Abtrennung der Zellstofffaser in dem ersten Schritt nicht abgetrennt werden konnte, zu einem Verzuckerungstank einschließt, um Glucose zu erhalten.

4. Verfahren nach Anspruch 3, das weiter einen sechsten Schritt der Fermentierung der Glucose, die in dem fünften Schritt erhalten wurde, einschließt, um Ethanol oder Milchsäure zu erhalten.

5. Verfahren nach einem der Ansprüche 1 bis 4, das weiter, vor dem ersten Schritt, einen Vorbehandlungswaschschritt einschließt, in dem die benutzten absorbierenden Artikel gewaschen werden.

6. Verfahren nach Anspruch 5, wobei Wasser, das aus der mikrobiellen Brennstoffzelle abgegeben wird, zu dem Vorbehandlungswaschschritt zurückgeführt wird.

7. Verfahren nach einem der Ansprüche 1 bis 6, wobei der elektrische Strom, der durch den dritten Schritt erhalten wird, zu mindestens einem Schritt unter folgenden zugeführt wird: dem ersten Schritt, dem zweiten Schritt, dem vierten Schritt, dem fünften Schritt, dem sechsten Schritt, dem Schritt der Rückgewinnung von superabsorbierendem Polymer und dem Vorbehandlungswaschschritt.

8. Verfahren nach einem der Ansprüche 1 bis 7, wobei der Festbrennstoff, der durch den zweiten Schritt erhalten wird, als Brennstoff für die Durchführung von mindestens einem Schritt unter folgenden verwendet wird: dem ersten Schritt, dem dritten Schritt, dem vierten Schritt, dem fünften Schritt, dem sechsten Schritt, dem Schritt der Rückgewinnung von superabsorbierendem Polymer und dem Vorbehandlungswaschschritt.

9. Verfahren nach einem der Ansprüche 1 bis 8, wobei eine Menge von Abfallmaterial, die durch das Verfahren erzeugt wird, weniger als 10 Masse-% einer Menge von benutzten absorbierenden Artikeln, die behandelt wurden, beträgt.

10. Verfahren nach einem der Ansprüche 1 bis 9, wobei ein Durchsatzverhältnis in dem ersten Schritt, dem zweiten Schritt und dem dritten Schritt entsprechend einem Bedarf für einen Zweck der Wiederverwendung verändert wird.

## Revendications

1. Procédé de recyclage d'un article absorbant usagé contenant des déchets humains, l'article absorbant comportant des matières constitutives comprenant de la fibre de pâte à papier et du polymère à fort pouvoir absorbant, le procédé comprenant :
une première étape consistant à séparer la fibre de pâte à papier de l'article absorbant usagé contenant des déchets humains, pour obtenir une fibre de pâte à papier régénérée, la première étape générant des eaux usées contenant des matières organiques,
une deuxième étape consistant à effectuer une conversion en combustible solide à partir des matières constitutives de l'article absorbant qui restent après la séparation de la fibre de pâte à papier, pour obtenir un combustible solide, et
une troisième étape consistant à fournir les eaux usées contenant des matières organiques générées au cours de la première étape à une pile à combustible microbienne pour obtenir de l'énergie électrique tout en purifiant les eaux usées ;
dans lequel un taux de traitement pour l'article absorbant usagé contenant des déchets humains est de 90 % en masse ou plus, le taux de traitement étant ((la quantité chargée de l'article absorbant usagé contenant des déchets humains) - (la quantité finale de déchets solides traités))/(la quantité chargée de l'article absorbant usagé contenant des déchets humains) × 100 ;
**caractérisé en ce que** le procédé comprend par ailleurs l'étape consistant à récupérer le polymère à fort pouvoir absorbant.

2. Procédé selon la revendication 1, comprenant par ailleurs une quatrième étape consistant à récupérer un sel nutritif en provenance des déchets humains.

3. Procédé selon la revendication 1 ou la revendication 2, comprenant par ailleurs une cinquième étape consistant à fournir les eaux usées contenant la fibre de pâte à papier n'ayant pas pu être séparée après la séparation de la fibre de pâte à papier au cours de la première étape, jusque dans un réservoir de saccharification pour obtenir du glucose.

4. Procédé selon la revendication 3, comprenant par ailleurs une sixième étape consistant à faire fermenter le glucose obtenu au cours de la cinquième étape pour obtenir de l'éthanol ou de l'acide lactique.

5. Procédé selon l'une quelconque des revendications 1 à 4, comprenant par ailleurs, avant la première étape, une étape de lavage de prétraitement au cours de laquelle les articles absorbants usagés sont lavés.

6. Procédé selon la revendication 5, dans lequel l'eau déchargée en provenance de la pile à combustible microbienne est renvoyée à l'étape de lavage de prétraitement.

7. Procédé selon l'une quelconque des revendications 1 à 6, dans lequel l'énergie électrique obtenue au cours de la troisième étape est fournie à au moins une étape parmi la première étape, la deuxième étape, la quatrième étape, la cinquième étape, la sixième étape, l'étape consistant à récupérer le polymère à fort pouvoir absorbant et l'étape de lavage de prétraitement.

8. Procédé selon l'une quelconque des revendications 1 à 7, dans lequel le combustible solide obtenu au cours de la deuxième étape est utilisé comme combustible pour la réalisation d'au moins une étape parmi la première étape, la troisième étape, la quatrième étape, la cinquième étape, la sixième étape, l'étape consistant à récupérer le polymère à fort pouvoir absorbant et l'étape de lavage de prétraitement.

9. Procédé selon l'une quelconque des revendications 1 à 8, dans lequel une quantité de déchets générés par le procédé est inférieure à 10 % en masse d'une quantité d'articles absorbants usagés qui ont été traités.

10. Procédé selon l'une quelconque des revendications 1 à 9, dans lequel un rapport de capacité au cours de la première étape, de la deuxième étape et de la troisième étape est changé en fonction d'une demande pour un objectif de réutilisation.
